# EUROPEAN PATENT APPLICATION

(11) **EP 4 847 053 A1**
(43) Date of publication of application: **02.12.2026**
(21) Application number: 26179124.8
(22) Date of filing: 18.05.2026
(51) Int. Cl.: A61M 1/02, A61B 90/98, A61J 1/10, G06K 17/00, G16H 40/20

(54) **METHODS AND SYSTEMS FOR PROCESSING BLOOD AND IDENTIFYING BLOOD PROCESSING CONTAINERS**

(30) Priority: 19.05.2025 US 202563808184 P
(71) Applicant: Fenwal, Inc., Lake Zurich, IL 60047 (US)
(72) Inventor: MADSEN, James Gregory, Lake Zurich, 60047 (US); KUSTERS, Benjamin, Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

Methods and systems for processing blood and identifying blood processing containers (12). In particular, a method and system for identifying containers used in a blood processing procedure. More particularly, methods and systems including an RFID system (26) to label a processing kit (14). More particularly, methods, systems, and devices including an RFID system to execute a blood processing procedure.

## Description

### FIELD OF THE DISCLOSURE

The present disclosure is directed to systems and methods for processing blood and identifying blood processing containers. More particularly, the present disclosure is directed to blood processing systems and methods including radiofrequency identification (RFID) devices. Even more particularly, the present disclosure is directed to blood processing systems and methods configured to use RFID devices to identify blood processing containers and to execute blood processing procedures.

### BACKGROUND

Whole blood can be collected from donors using manual collection procedures through blood drives, donor visits to blood centers or hospitals. Blood drives can occur at various locations such as schools, workplaces, buses, and local blood collection centers. During collection, a volume of a donor's whole blood can be collected in a collection container (e.g., a flexible pouch or bag). Generally, the collection containers can include a set of labels (e.g., about four or five labels) to identify the collection container and any subsequent processing containers associated with the collection container. For instance, the labels can include the donor's identification number (DIN). The DIN and any other information can be written on or printed onto the labels.

Typically, the collection container is pre-attached to a processing kit/circuit. The processing kit can include a number of processing containers and filters used for processing the whole blood that has been separated into its constituent components, such as red blood cells (RBCs), plasma, and buffy coat. The pre-attached processing kit can be configured to be used for a particular blood processing procedure, such as RBC collection.

Alternatively, a single collection container can be sent out for collections in the field, and the appropriate processing kit can be sterilely docked to the collection container at a processing center. This approach reduces the volume of processing kits sent out for collections (one container versus four or more containers) and allows blood processing centers to decide on the best processing method after all collections have been made, thereby providing more flexibility.

Currently, to maintain and track donor identification information, a technician (e.g., the person collecting blood from the donors or technician at the processing center), may manually remove one of the labels including the DIN (and other information) from the collection container and attach it to the processing containers. This process can be tedious and prone to errors, such as stickers becoming detached or being misplaced.

Additionally, in traditional whole blood processing, whole blood is manually separated into its components - RBCs, plasma, and buffy coat. This process involves loading a collection container with whole blood into a centrifuge to separate the whole blood into its components and manually expressing the separated components using an expressor operated by an experienced technician. The operator must visually monitor and determine when to stop expressing the spun whole blood units, often managing 6-8 expressors simultaneously. This manual approach can lead to inconsistencies and suboptimal separation of blood components.

There exists a need for improved systems and methods for identifying blood processing containers and for processing whole blood.

### SUMMARY

In one aspect, a system for identifying containers used in a blood processing procedure is provided. The system includes a collection container including a first permanent RFID tag, a sterile docking station, and an RFID system associated with the sterile docking station. The system also includes a disposable blood processing kit. The collection container is configured to be sterilely connected to the disposable blood processing kit and the RFID system is configured to label the blood processing kit with information from the first permanent RFID tag.

In another aspect, a system for processing whole blood is provided. The system includes a collection container associated with a disposable blood processing kit. The disposable blood processing kit includes an RFID tag. The system also includes a reusable blood processing device configured to receive at least the disposable blood processing kit. Additionally, the system includes an RFID reader associated with the reusable processing device. The RFID reader is configured to read information on the RFID tag. The system also includes a controller associated with the reusable blood processing device and coupled to the RFID reader. The controller is configured to automatically execute one or more blood processing procedures based on the information on the RFID tag.

In another aspect, a method for identifying a blood processing kit and processing whole blood is provided. The method includes connecting, in a sterile manner, a collection container containing whole blood to a blood processing kit. The collection container includes a first permanent RFID tag. The method further includes labelling the blood processing kit with an RFID system based on the first permanent RFID tag on the collection container.

### BRIEF DESCRIPTION OF THE DRAWINGS

FIG. 1 is a plan view of an example of a collection container including an RFID tag and a processing kit associated with a sterile docking station.
FIG. 2 is a plan view of another example of a collection container and a processing kit associated with a sterile docking station.
FIG. 3 is a plan view of another example of a collection container and processing kit associated with a sterile docking station.
FIG. 4 is a plan view of yet another example of a collection container and processing kit associated with a sterile docking station.
FIG. 5 is a plan view of a labeled processing kit associated with a processing device.

### DETAILED DESCRIPTION OF THE EMBODIMENTS

A more detailed description of the systems and methods in accordance with the present disclosure is set forth below. It will be understood that the description below of specific devices and methods is intended to be exemplary, and not exhaustive of all possible variations or applications.

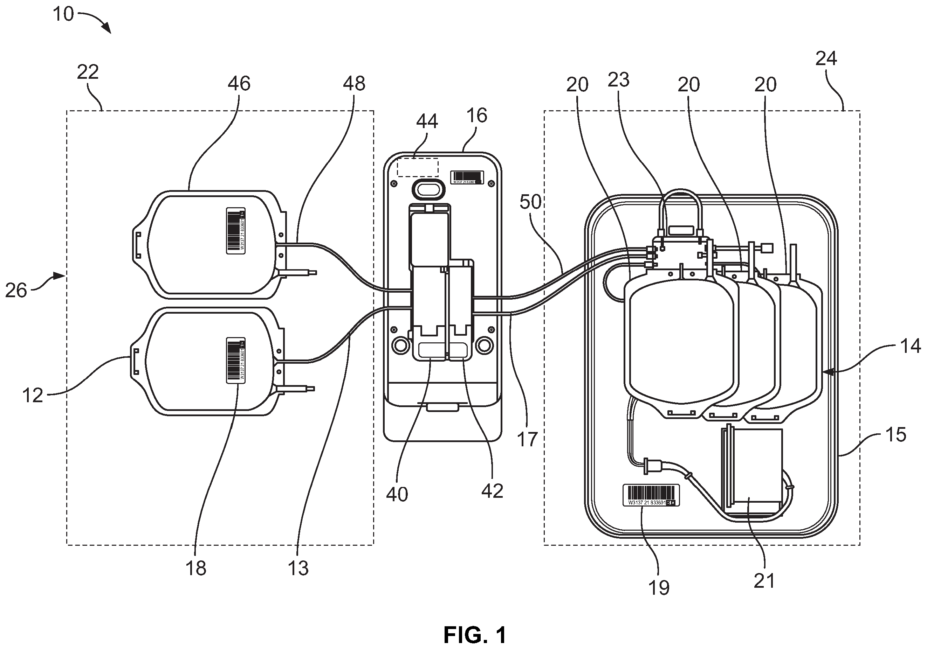

FIG. 1 shows an example of a system 10 for identifying containers used in whole blood collection and a blood processing procedure. The system 10 includes a whole blood collection container 12, a disposable processing kit 14, and a sterile docking station 16. In an example, system 10 is configured to automatically label the processing kit 14. For instance, the system 10 is configured to automatically transfer information from the collection container 12 to the processing kit 14.

Collection container 12 can be filled with whole blood collected from a donor. For example, whole blood can be collected from a donor in the collection container 12 at various locations, such as, but not limited to, schools, workplaces, buses, and local blood collection centers. The collection container 12 can be formed of a material suitable to contain whole blood such as plasticized polyvinyl chloride (PVC) or a non-PVC polyolefin. The collection container 12 can include outlet tubing 13 configured to be connected in a sterile manner to a processing kit 14.

Often, whole blood will be collected from multiple donors at once and/or during a period of time from a blood collection technician. To facilitate multiple whole blood collections, multiple collection containers 12 can be transported to the location of the collection.

The collection container 12 can include labels to identify the collected blood and the whole blood source (e.g., donor). In an example, collection container 12 includes a permanent RFID tag 18. The RFID tag 18 can be pre-attached to the surface of the collection container 12. For example, the RFID tag 18 can be permanently attached to the collection container 12 by adhesion, welding, bonding, or any other suitable permanent attachment. The RFID tag 18 can be configured to include various information, including, but not limited to a donor identification number (DIN). Other useful donor information such as height, weight, sex, age, and collection information such as volume, blood type, etc., can be encoded to the RFID tag 18 before, during, or after collection.

After whole blood collection, the filled collection container(s) 12 can be transported to a processing facility, where they can be sterilely attached to a disposable blood processing kit 14 to undergo various blood processing procedures. The processing kit 14 can include at least one processing container 20. In some examples the blood processing kit 14 can include a plurality of processing containers 20. The blood processing kit can include a separation chamber 21. Additionally, in some examples, the processing kit 14 can include a processing cassette 23 configured to include various stations (e.g., valve stations and pump stations) and flow paths to carry out fluid processing procedures implemented by a fluid processing device. The cassette 23 can be associated with various valves and pumps of a reusable fluid processing device 30 to direct flow through the processing kit 14.

The processing kit 14 includes tubing defining flow paths placing the processing containers 20 in flow communication with and between the various components of the processing kit 14, such as the cassette 23 and the separation chamber 21. In particular, the processing kit 14 includes inlet tubing 17 configured to be sterilely connected to the collection container 12, to place the collection container 12 and processing kit 14 in fluid communication with each other.

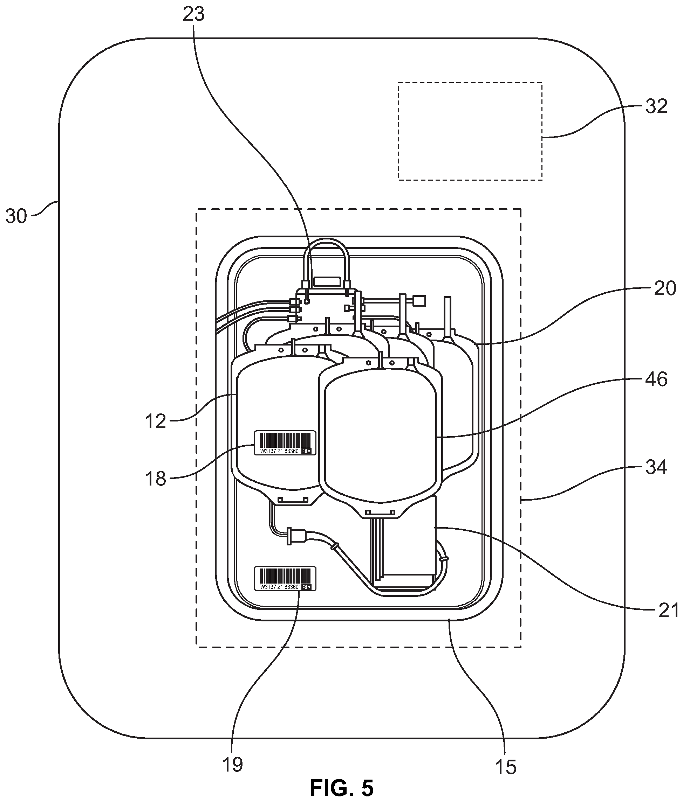

In an example, as shown in FIGS. 1-5, the processing kit 14 can include a tray 15 or other receptacle for housing the processing containers 20 and other components of processing kit 14 prior to use. For instance, the processing containers 20 can be assembled on tray 15 to provide processing kit 14 as a compact unit. Alternatively, processing kit 14 can be packaged without a tray 15.

The processing kit 14 can be configured to be used in a particular blood processing procedure. For instance, the processing kit 14 can include various processing containers 20, such as in-process containers, additive solution containers, and/or separation containers for use in a particular blood processing procedure. In an example, processing kit 14 can include processing containers 20 configured for a procedure to separate whole blood into red blood cells (RBCs) and plasma. In another example, the processing kit 14 can include processing containers 20 configured for a procedure to separate whole blood into RBCs, plasma, and a buffy coat. In yet another example, the processing kit 14 can include processing containers 20 configured for a procedure to pool buffy coats from multiple whole blood units for further processing to create platelet units. The processing kit 14 can include any suitable configuration of processing containers 20 and other components for any suitable blood processing procedure.

After collection of whole blood from a donor, the filled collection container 12 can be attached to the processing kit 14 via docking in a sterile manner. In particular, outlet tubing 13 of the collection container 12 can be sterilely connected to inlet tubing 17 of the processing kit 14, thus placing the collection container 12 in fluid communication with the processing kit 14.

In an example, the collection container 12 and the processing kit 14 can be connected to one another via a sterile docking station 16. For instance, the collection container 12 and the processing kit 14 can be connected to each other using a sterile docking station as described in U.S. Patent No. 11,731,371 issued August 22, 2023, which is incorporated in its entirety herein. In an example, the sterile docking station 16 includes a first carriage 40 and a second carriage 42. The first carriage 40 can be configured to receive the collection container 12 and the second carriage 42 can be configured to receive the processing kit 14. In particular, the first carriage 40 can define a first portion of a slot configured to receive outlet tubing 13 of the collection container 12, while the second carriage 42 defines a second portion of the slot configured to receive inlet tubing 17 of the processing kit 14. The sterile docking station 16 includes a controller 44 configured to execute a sterile connection procedure. For example, to sterilely connect the collection container 12 to the processing kit 14, a solid cutting blade is heated, followed by the heated blade being moved to a cutting position to cut sealed outlet tubing 13 and inlet tubing 17 received by the slot. The second carriage 42 can move parallelly with respect to the first carriage 40 so as to align the cut ends of tubing 13 and 17. The heated blade then moves out of the cutting position, followed by the first carriage 40 moving toward the second carriage 42 so as to press the cut ends of tubing 13 and 17 into contact with each other so as to sterilely connect the cut ends and define a joined tube.

Optionally, depending on the configuration of the processing kit 14 and requirements for a particular blood processing procedure, an auxiliary container 46, which can contain an additive fluid (e.g., saline, anticoagulant, or other suitable additive fluid) can also be attached to the processing kit 14 in the manner described herein. In some instances, the sterile docking station 16 can include multiple slots defined within the carriages 40 and 42 and is configured to sterilely connect multiple containers at once. For instance, as shown in FIG. 1, an auxiliary container 46 includes outlet tubing 48, which can be sterilely connected to a second inlet tubing 50 of the processing kit 14.

Additionally, in accordance with the present disclosure, the sterile docking station 16 may be associated with an RFID system 26. The RFID system 26 may include one or more RFID devices configured to read RFID information from RFID tags associated with the collection container 12 and/or processing kit 14. The RFID system 26 is also configured to write/encode information onto RFID tags.

In an example, the RFID system 26 can be integral with the docking station 16. Alternatively, the RFID system 26 can be independent from the sterile docking station 16. For example, the sterile docking station 16 can be a table-top device with the RFID system located in proximity to the collection container 12 and processing kit 14 in a way that permits non-interfered radio wave transmission and/or communication between an RFID device and the tags on the containers. In one embodiment, RFID system 26 can be mounted under the table below the sterile docking station 16. In another embodiment, the RFID system 26 can be located adjacent to or above the sterile docking station 16. The RFID system 26 can also be coupled to a computing device associated with the identification system 10, a controller 44 associated with the sterile docking station 16, and/or a controller 32 associated with a blood processing system/device 30 as described in greater detail herein.

In one example, the RFID tags and devices can operate at 13.56 MHz although it will be understood that the RFID tags and devices can operate at different frequencies including, in another non-limiting example, 125 MHz. Additionally, the RFID tags and devices can operate at any suitable detection range including, for example, a detection range of about 1-2 meters. Examples of suitable RFID tags include, but are not limited to, RFID Tag RF700069 available from Avery Dennison and RFID Tag FI-I11-114A-S1 available from Texas Instruments. Suitable RFID transponders include, but are not limited to, the RFID Reader 13.56 MHz Module DLP-RFID1-OG available from DLP Design Inc. and the RFID Reader 13.56 MHz Module RI-STU-TRDC-30 available from Texas Instruments.

The RFID system 26 can include at least one RFID device configured to read RFID information and/or encode RFID information onto an RFID tag. For instance, the RFID system 26 can include an RFID device located on either side of the sterile docking station 16. For example, as shown in FIGS. 1-4, a first RFID device 22 can be located on one side of the sterile docking station 16 and a second RFID device 24 can be located on the opposite side of the sterile docking station 16. In this configuration, the first RFID device 22 can read and encode RFID tags associated with the collection container 12 (and optional auxiliary containers 46) and the second RFID device 24 can read and encode RFID tags associated with the processing kit 14. Alternatively, the RFID system 26 includes one RFID device, which can read and encode RFID tags located on either side of the sterile docking station 16. RFID tags may be passive or active.

The RFID system 26 is configured to assist in identifying the various containers associated with a blood processing system/procedure and in executing a particular blood processing procedure. In particular, the RFID system 26 is configured to label the processing kit 14 and the containers and other components thereof. For instance, the RFID system 26 is configured to read and encode information from and to RFID tags associated with a collection container 12 and a processing kit 14. In an example, the RFID system 26 is configured to transfer RFID information from a collection container 12 to a processing kit 14 and/or the components thereof. RFID information can be transferred from an RFID tag associated with a collection container 12 automatically or manually by a technician. Additionally, the RFID information encoded on an RFID tag associated with either a collection container 12 and/or a processing kit 14 can be used to identify a particular procedure to be executed using the processing kit 14.

Turning to FIGS. 1-4, the figures illustrate examples of system 10 for identifying blood processing containers. As described herein, a collection container 12 can include a permanent RFID tag 18. In an example, the RFID tag 18 can be pre-attached to the collection container 12. The RFID tag 18 can be encoded with donor information, including, but not limited to a DIN. In an example, the RFID tag 18 can be pre-encoded with an identification number before being deployed for collection or it can be encoded with donor information, including an identification number, at the donation location. In some examples, additional information can be encoded onto RFID tag 18.

After collection container 12 is filled with whole blood, the filled collection container 12 can be connected to a processing kit 14 via a sterile docking station 16 as described herein. Before sterilely connecting the collection container 12 and processing kit 14, the RFID system 26 can verify that the RFID information on the RFID tag 18 has not already been used or flagged. For instance, an RFID device can read and identify the DIN encoded on the RFID tag 18 attached to the collection container 12 and send the information to a computing device including a data management system. The information from the RFID tag 18 can be cross-referenced with the data management system to verify that the DIN has not already been used or designated as processed, or that there are no other warnings or errors associated with the information.

If the information is verified (e.g., the DIN has not yet been used or designated as processed), the RFID system 26 can label the processing kit 14 based on the information on the RFID tag 18. In an example, the tray 15 (if included) can be labeled and/or the processing containers 20 included in processing kit 14 can be labeled. For instance, encoded information on the RFID tag 18 can be copied onto additional RFID tags 19 or directly onto the processing kit 14.

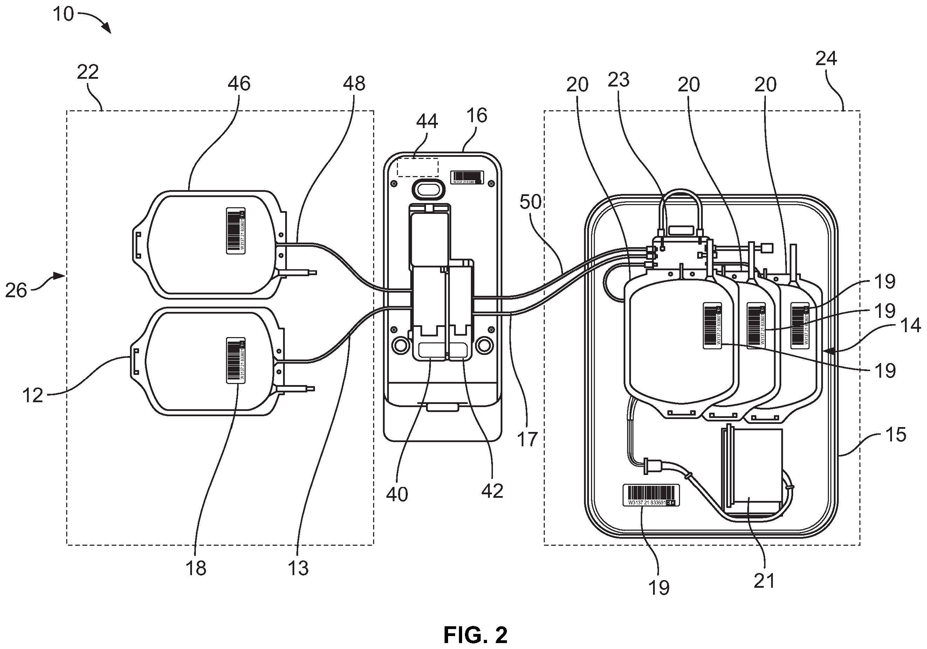

In an example, as shown in FIG. 2, RFID system 26 can generate additional RFID tags 19 for the processing kit 14 and/or processing containers 20. For instance, the processing kit 14 can include at least one additional pre-attached RFID tag 19. The at least one additional RFID tag 19 can include a single additional RFID tag 19 attached to the processing kit 14, such as to the tray 15 or to a processing container 20. Alternatively, the system 10 can include a plurality of additional RFID tags 19 attached to multiple processing containers 20 and/or the tray 15 (if present). In an example, as shown in FIG. 2, each processing container 20 and the tray 15 can include an additional RFID tag 19.

After verification of the RFID tag 18 attached to the collection container 12, the RFID system 26 can encode at least one additional pre-attached RFID tag 19 based on the information encoded on the RFID tag 18 on the collection container 12. For instance, the information on the RFID tag 18 can be copied onto at least one additional pre-attached RFID tag 19.

In another example, the RFID system 26 can print, encode, or otherwise generate new additional RFID tags 19 to be placed on the processing kit 14. For instance, RFID system 26 can include a printer, and after verification of the RFID tag 18 attached to the collection container 12, the RFID system 26 can print and encode new additional RFID tags 19 based on the information encoded on the RFID tag 18 on the collection container 12. The RFID system 26 can be configured to automatically attach the new additional RFID tags 19 to the processing kit 14 or the new tags 19 can be manually attached to the processing kit 14 by a technician.

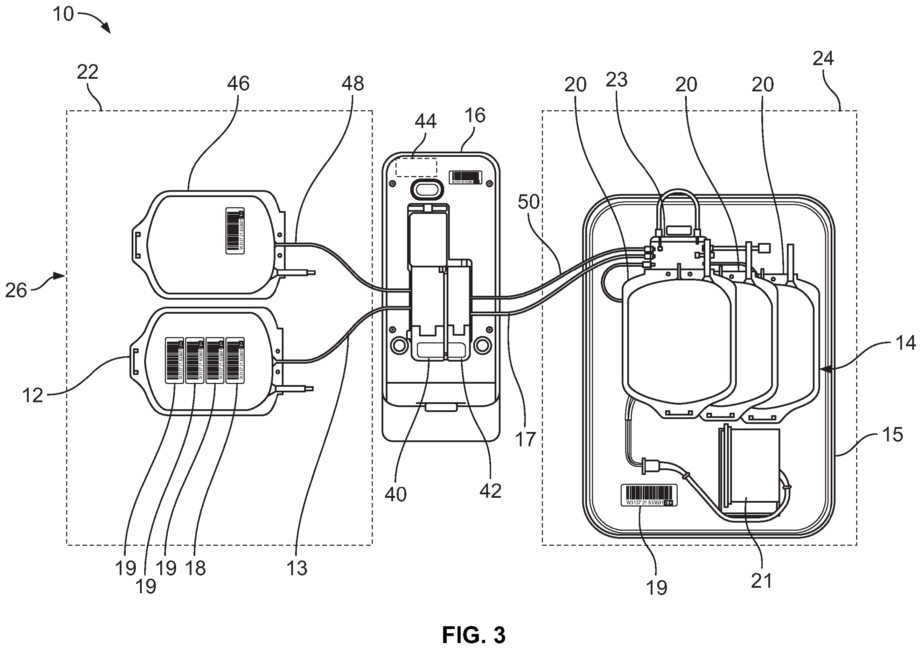

FIG. 3 illustrates another example of labeling the processing kit 14. In addition to the pre-attached RFID tag 18, the collection container 12 can also include at least one pre-attached additional RFID tags 19. The pre-attached additional RFID tags 19 can be removably adhered to the collection container 12. In another example, the pre-attached additional RFID tags 19 can be temporarily attached using any other suitable temporary attachment mechanism.

As shown in FIG. 3, the collection container 12 can include a plurality of additional RFID tags 19 for the processing kit 14. For example, the collection container 12 can include an additional RFID tag 19 for multiple processing containers 20 and/or the tray 15 (if present). In a particular example, the collection container 12 can include an additional RFID tag 19 for the tray 15 and for each processing container 20. The number of additional RFID tags 19 provided on collection container 12 can vary without departing from the scope of the disclosure. After verification of the RFID tag 18 permanently attached to the collection container 12, the RFID system 26 can encode the additional RFID tags 19 based on the information encoded on the RFID tag 18. A technician can manually transfer the additional RFID tags 19 from the collection container 12 to the processing kit 14. The additional RFID tags 19 can be encoded by the RFID system 26 either before being transferred from the collection container 12 or after being attached to the processing kit 14.

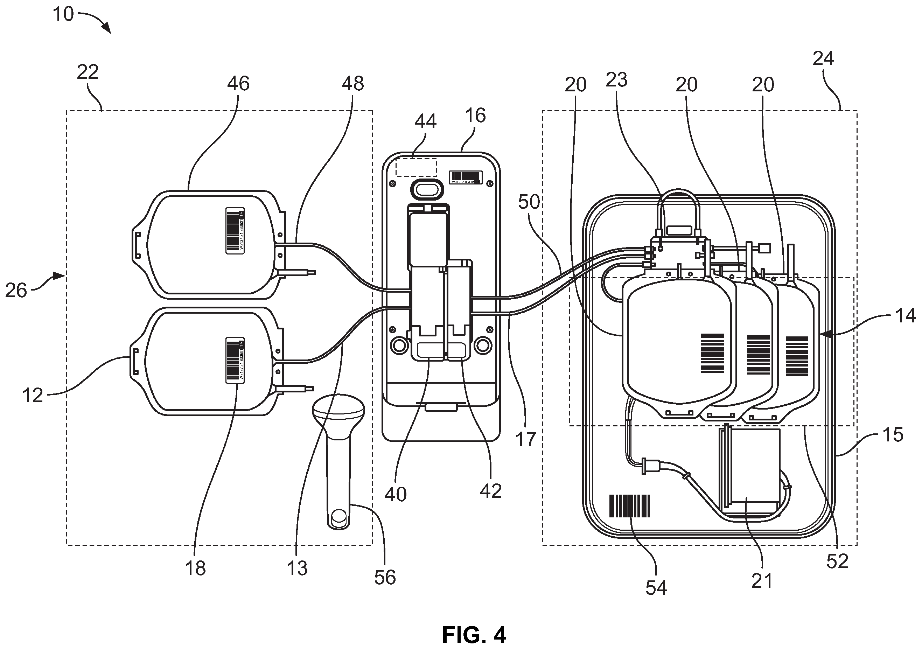

Turning to FIG. 4, the figure illustrates another example of labeling the processing kit 14. In an example, the RFID system 26 can be associated with a laser printer 52. For instance, the RFID system 26 can be coupled to a laser printer (e.g., via a wireless or wired connection). The laser printer can be located and configured to print directly onto the processing kit 14. In an example, the printer can print directly onto the tray 15 (if present) and/or onto the processing containers 20. After verification of the RFID tag 18 attached to the collection container 12, the RFID system 26 can direct the printer to print information directly on the processing kit 14. For instance, the printer can print the information from the RFID tag 18 on the collection container 12 onto the processing kit 14 in the form of a barcode 54 or QR code, or it can print the information alpha-numerically. The information can be printed onto the components of the processing kit 14 in various formats or symbols without departing from the scope of the disclosure.

After labelling the processing kit 14, the RFID system 26 can designate the RFID tag 18 on the collection container 12 as "processed." For instance, the RFID system 26 can send a signal to the computing device to designate that the RFID tag 18 on the collection container 12 has been processed. The RFID system 26 can verify that the additional RFID tags 19 on the processing kit 14 have been correctly labeled. For instance, the RFID system 26 can read the additional RFID tags 19 to ensure that the information matches the information on the RFID tag 18 attached to the collection container 12. When the RFID system 26 laser printed the information onto processing kit 14, a hand-held barcode reader 56 (FIG. 4) can be used to confirm that the information was properly laser printed onto the processing kit 14.

The collection container 12 and the processing kit 14 can be sterilely attached to one another before or after labelling the processing kit 14. In an example, the RFID system 26 can label the processing kit 14 as described herein and after verifying that the processing kit 14 has been properly labeled, the RFID system 26 can send a signal to sterile docking station 16 to sterilely connect the collection container 12 to the processing kit 14. In another example, the collection container 12 can be sterilely docked to the processing kit 14 before the RFID system 26 labels the processing kit 14. For instance, the RFID system 26 can verify that the RFID information on RFID tag 18 has not been designated as processed. Then the sterile docking station can sterilely connect the collection container 12 to the processing kit 14. After sterile connection, the RFID system 26 can label the processing kit 14. In yet another example, the collection container 12 can be sterilely docked to the processing kit 14 simultaneously with the RFID system labelling the processing container.

By tracking the collection container 12 and labelling the processing kit 14 with system 10, a fluid circuit (including the collection container 12 and processing kit 14) can be tracked without the need for handheld scanning. Information transfers from RFID tags 18 to the additional RFID tags 19 can be confirmed automatically and the collection container 12 and processing kit 14 can be tracked anywhere within a blood center, improving the traceability of each component. Additionally, in some instances, by employing system 10 collection containers 12 can be sent to the collection locations with only one RFID tag 18, thus preventing the risk of mishandling additional labels or tags located on the collection container 12.

It will be understood that identification system 10 can be employed with any procedure requiring the sterile docking of containers. For instance, in addition to the procedures discussed above, system 10 can be employed for sterile docking additional containers to a processing kit to split an apheresis unit into multiple units or for sterile docking containers to create cryo-precipitate pools from frozen plasma.

After sterile connection and labelling the processing kit 14, the connected collection container 12 and processing kit 14 can be loaded onto a reusable processing device 30 to undergo a blood processing procedure. As described above, the processing kit can be configured to be executed with a variety of fluid processing procedures. For example, the processing kit 14 can be configured for an RBC and plasma separation; a RBC, plasma, and buffy coat separation; a procedure pooling buffy coats from multiple whole blood units for further processing to collect platelet units, and any other suitable blood processing procedures. The blood processing procedures can be executed by a variety of reusable blood processing devices 30, including, but not limited to the Alyx, Amicus, and Amicore systems provided by Fenwal, Inc. of Lake Zurich, IL.

In an example, the blood processing device 30 can include a separator configured to separate whole blood into its constituent components. In an example, the separator can be configured to receive the collection container 12 or the processing kit 14 can include a separation chamber configured to be received by the separator.

Additionally, the blood processing device 30 can include a controller 32 configured to execute one or more blood processing procedures. The controller 32 can be integral with the blood processing device or it can be an external controller 32. The blood processing device 30 can be associated with an RFID device 34. For instance, the RFID device 34 can be coupled to the controller 32. In an example, the RFID device 34 can be integral with the reusable blood processing device 30. In another example, the RFID device 34 can be a separate device located near the reusable blood processing device 30. In particular, the RFID device 34 can be coupled, wirelessly or via a wired connection, to controller 32 of the reusable blood processing device 30.

An RFID tag on the processing kit 14 can be encoded with blood processing procedure information associated with the processing kit 14. For instance, the blood processing procedure information can include, but is not limited to, the type of procedure to be executed with the processing kit 14; processing kit lot, serial number, and expiration date; additive solution lot, serial number, and expiration date; and whole blood data including DIN and volume. In an example, the blood processing procedure information can be encoded onto an additional RFID tag 19 as described herein. In another example, the processing kit 14 can include a separate pre-attached processing kit RFID tag including the blood processing procedure information. In an example, the separate pre-attached processing kit RFID tag can be permanently attached to the processing kit 14.

The RFID device 34 is configured to read the RFID tag(s) associated with the processing kit 14 and to send the information to the controller 32. Based on the blood processing procedure information, the controller 32 can direct the various pumps and valves of the reusable blood processing device 30 to execute a blood processing procedure.

To use the reusable blood processing device 30, the processing kit 14 (after being sterilely connected to the collection container 12) is placed onto the device 30. The RFID device 34 can then read the RFID information associated with the processing kit 14, including the blood processing procedure information, and can send the information to the controller 32. The controller 32 can then automatically execute a blood processing procedure based on the blood processing procedure information from the RFID device 34.

In an example, controller 32 can be coupled to a computing device including a data management system, such as the computing device described herein, and can be configured to send blood processing procedure information/data to the computing device. For instance, the reusable blood processing device 30 can include sensors and trackers configured to track characteristics and data on the blood processing procedure. In an example, the sensors and trackers can include optical sensors, pressure sensors, and any other suitable sensors without departing from the scope of the disclosure. Information from the sensors and trackers can be sent to the controller 32. The controller 32 can be configured to store the information and to send the information to a computing device.

Using RFID tags associated with the processing kit 14 and an RFID device 34, the blood processing procedure can be executed with minimal operator intervention, allowing for a more streamlined procedure. For instance, by using RFID device 34, there is a reduction on the dependency of handheld technology such as RFID scanners and data can automatically be sent to the controller 32 and from the controller 32 to a data management system. Additionally, the use of RFID device 34 can allow for future integration with robotic systems for further automation.

In an example, a single RFID device can be used with the identification system 10, labelling the processing kit 14, and with the reusable blood processing device 30. Alternatively, the identification system 10 and blood processing device 30 can include separate RFID devices.

There are additional aspects to the devices and methods described herein including, without limitation the following aspects.

Aspect 1. A system for identifying containers used in a blood processing procedure including a collection container including a first permanent RFID tag; a sterile docking station; an RFID system associated with the sterile docking station; a disposable blood processing kit, wherein the collection container is configured to be connected in a sterile manner to the disposable blood processing kit; and wherein the RFID system is configured to label the blood processing kit with information from the first permanent RFID tag.

Aspect 2. The system of Aspect 1, wherein the disposable blood processing kit includes one or more processing containers.

Aspect 3. The system of Aspect 2, wherein the disposable blood processing kit includes a tray including the one or more processing containers.

Aspect 4. The system of any one of Aspects 1-3, wherein the RFID system includes an RFID device configured to read and to write RFID information.

Aspect 5. The system of any one of Aspects 1-4, wherein the RFID system is associated with a computing device.

Aspect 6. The system of any one of Aspects 1-5, wherein the RFID system is configured to verify that the first permanent RFID tag has not been processed.

Aspect 7. The system of any one of Aspects 1-6, wherein the sterile docking station includes a first carriage configured to receive the collection container and a second carriage configured to receive the disposable blood processing kit.

Aspect 8. The system of any one of Aspects 1-7, wherein the RFID system is configured to generate additional RFID tags for the disposable blood processing kit, wherein the additional RFID tags include information from the first permanent RFID tag on the collection container.

Aspect 9. The system of any one of Aspects 1-7, wherein the collection container includes additional RFID tags, wherein the additional RFID tags are configured to be manually placed on the disposable blood processing kit.

Aspect 10. The system of Aspect 9, wherein the RFID system is configured to copy the information from the first permanent RFID tag to the additional RFID tags.

Aspect 11. The system of any one of Aspects 1-7, comprising a printer associated with the RFID system and configured to print information from the first permanent RFID tag onto the disposable blood processing kit.

Aspect 12. A system for processing whole blood including a collection container associated with a disposable blood processing kit, wherein the disposable blood processing kit includes an RFID tag; a reusable blood processing device configured to receive at least the disposable blood processing kit; an RFID device associated with the reusable blood processing device, wherein the RFID device is configured to read information on the RFID tag; and a controller associated with the reusable blood processing device and coupled to the RFID device, wherein the controller is configured to automatically execute one or more blood processing procedures based on the information on the RFID tag.

Aspect 13. The system of Aspect 12, wherein the disposable blood processing kit includes one or more processing containers.

Aspect 14. The system of any one of Aspects 12-13, wherein the reusable blood processing device includes a separator configured to receive a collection container including whole blood and to separate the whole blood into its constituent components.

Aspect 15. The system of any one of Aspects 12-14, wherein the one or more blood processing procedures includes a blood processing procedure separating whole blood into a red blood cell component and a plasma component and wherein the disposable processing kit is configured to be used in the blood processing procedure separating whole blood into a red blood cell component and a plasma component.

Aspect 16. The system of any one of Aspects 12-14, wherein the one or more blood processing procedures includes a blood processing procedure separating whole blood into a red blood cell component, plasma component, and a buffy coat, and wherein the disposable processing kit is configured to be used in the blood processing procedure separating whole blood into a red blood cell component, plasma component, and a buffy coat.

Aspect 17. The system of any one of Aspects 12-14, wherein the one or more blood processing procedures includes a blood processing procedure pooling buffy coats from multiple whole blood units to be processed to collect platelet units and wherein the disposable blood processing kit is configured to be used in the blood processing procedure pooling buffy coats from multiple whole blood units to be processed to collect platelet units.

Aspect 18. The system of any one of Aspects 12-17, wherein the information on the RFID tag includes blood processing procedure information.

Aspect 19. A method for identifying a blood processing kit and processing whole blood including connecting in a sterile manner a collection container containing whole blood to a blood processing kit, wherein the collection container includes a first permanent RFID tag; and labelling the blood processing kit with an RFID system based on the first permanent RFID tag on the collection container.

Aspect 20. The method of Aspect 19, wherein the blood processing kit includes one or more processing containers.

Aspect 21. The method of any one of Aspects 19-20, including connecting in a sterile manner the collection container to the blood processing kit at a sterile docking station, wherein the RFID system is associated with the sterile docking station.

Aspect 22. The method of any one of Aspects 19-21, wherein the RFID system includes an RFID device configured to read and write RFID information.

Aspect 23. The method of any one of Aspects 19-22, wherein the sterile docking station includes a first carriage configured to receive the collection container and a second carriage configured to receive the blood processing kit.

Aspect 24. The method of any one of Aspects 19-23, including verifying that the first permanent RFID tag has not been processed.

Aspect 25. The method of any one of Aspects 19-24, including generating additional RFID tags with the RFID system and placing the additional RFID tags on the blood processing kit, wherein the additional RFID tags includes information from the first permanent RFID tag on the collection container.

Aspect 26. The method of any one of Aspects 19-24, wherein the collection container includes additional removeable RFID tags, and further including copying information from the first permanent RFID tag to the additional removeable RFID tags with the RFID system and placing the additional removeable RFID tags onto the one or more processing containers.

Aspect 27. The method of any one of Aspects 19-24, including printing information from the first permanent RFID tag onto the blood processing kit.

Aspect 28. The method of any one of Aspects 19-27, including processing the whole blood with a reusable blood processing device, wherein the reusable blood processing device is configured to automatically execute a blood processing procedure based on the labeled blood processing kit.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

## Claims

1. A system for identifying containers used in a blood processing procedure comprising:
a collection container including a first permanent RFID tag;
a sterile docking station;
an RFID system associated with the sterile docking station;
a disposable blood processing kit, wherein the collection container is configured to be connected in a sterile manner to the disposable blood processing kit; and
wherein the RFID system is configured to label the blood processing kit with information from the first permanent RFID tag.

2. The system of Claims 1, wherein the RFID system includes an RFID device configured to read and write RFID information.

3. The system of any one of Claims 1-2, wherein the RFID system is configured to verify that the first permanent RFID tag has not been processed.

4. The system of any one of Claims 1-3, wherein the RFID system is configured to generate additional RFID tags for the disposable blood processing kit, wherein the additional RFID tags include information from the first permanent RFID tag on the collection container.

5. The system of any one of Claims 1-4, wherein the collection container includes additional RFID tags, wherein the additional RFID tags are configured to be manually placed on the disposable blood processing kit.

6. The system of Claim 5, wherein the RFID system is configured to copy the information from the first permanent RFID tag to the additional RFID tags.

7. The system of any one of Claims 1-6, comprising a printer associated with the RFID system and configured to print information from the first permanent RFID tag onto the disposable blood processing kit.

8. A system for processing whole blood comprising:
a collection container associated with a disposable blood processing kit, wherein the disposable blood processing kit includes an RFID tag;
a reusable blood processing device configured to receive at least the disposable blood processing kit;
an RFID device associated with the reusable blood processing device, wherein the RFID device is configured to read information on the RFID tag; and
a controller associated with the reusable blood processing device and coupled to the RFID device, wherein the controller is configured to automatically execute one or more blood processing procedures based on the information on the RFID tag.

9. A method for identifying a blood processing kit and processing whole blood comprising:
connecting in a sterile manner a collection container containing whole blood to a blood processing kit, wherein the collection container includes a first permanent RFID tag; and
labelling the blood processing kit with an RFID system based on the first permanent RFID tag on the collection container.

10. The method of Claim 9, comprising connecting in a sterile manner the collection container to the blood processing kit at a sterile docking station, wherein the RFID system is associated with the sterile docking station.

11. The method of any one of Claims 9-10, comprising verifying that the first permanent RFID tag has not been processed.

12. The method of any one of Claims 9-11, comprising generating additional RFID tags with the RFID system and placing the additional RFID tags on the blood processing kit, wherein the additional RFID tags include information from the first permanent RFID tag on the collection container.

13. The method of any one of Claims 9-11, wherein the collection container includes additional removeable RFID tags, and further comprising copying information from the first permanent RFID tag to the additional removeable RFID tags with the RFID system and placing the additional removeable RFID tags onto the one or more processing containers.

14. The method of any one of Claims 9-11, comprising printing information from the first permanent RFID tag onto the blood processing kit.

15. The method of any one of Claims 9-14, comprising processing the whole blood with a reusable blood processing device, wherein the reusable blood processing device is configured to automatically execute a blood processing procedure based on the labeled blood processing kit.
